# EUROPEAN PATENT SPECIFICATION

(11) **EP 3 751 247 B1**
(45) Date of publication and mention of the grant of the patent: **18.12.2024**
(21) Application number: 19751623.0
(22) Date of filing: 11.02.2019
(51) Int. Cl.: G01L 1/04, A61B 18/12, A61B 18/00, A61M 25/00, G01L 1/22, A61B 5/0215, A61B 5/00, A61B 18/14, A61B 9/00, A61B 90/00

(54) **FORCE SENSOR, AND ELECTROPHYSIOLOGY CATHETER**
KRAFTSENSOR UND ELEKTROPHYSIOLOGISCHER KATHETER
CAPTEUR DE FORCE ET CATHÉTER ÉLECTROPHYSIOLOGIQUE

(30) Priority: 11.02.2018 CN 201810142142
(43) Date of publication of application: 16.12.2020
(73) Proprietor: Shanghai Microport Ep Medtech Co., Ltd., Shanghai 201318 (CN)
(72) Inventor: SHEN, Lei, Shanghai 201318 (CN); LIANG, Bo, Shanghai 201318 (CN); MIAO, Tao, Shanghai 201318 (CN); WANG, Hui, Shanghai 201318 (CN); WANG, Mei, Shanghai 201318 (CN)
(74) Representative: Patentship Patentanwaltsgesellschaft mbH
(86) International application number: PCT/CN2019/074760
(87) International publication number: WO 2019/154409

(56) References cited:
- CN-A- 102 375 586
- CN-A- 106 264 719
- CN-A- 106 264 719
- CN-A- 107 477 124
- CN-A- 107 496 055
- CN-U- 202 678 568

## Description

### TECHNICAL FIELD

The present invention relates to the technical field of medical instruments and, more specifically, to a force sensor and an electrophysiology catheter.

### BACKGROUND

In recent years, catheter systems have been developed for interventional treatment of, for example, cardiac arrhythmias and refractory hypertension. For example, in the treatment of atrial fibrillation, one type of cardiac arrhythmia, an ablation or mapping catheter may be introduced into the heart via a vein or artery to find an aberrant electrical signal position or pathway by endocardial mapping, and then apply energy at the position or pathway to ablate it to eliminate or alter undesirable electrical signals, thus achieving curative results. Another example is the treatment of refractory hypertension through renal artery ablation, in which an ablation catheter may be arterially introduced into an artery connecting the abdominal aorta and the kidney to ablate and block the parasympathetic nerve pathway to lower the blood pressure.

For ablation therapy, how strongly an electrode disposed on a distal end of the used catheter contacts the target vessel wall or tissue is considered very important. Weak contact will lead to a superficial ablation lesion, and is thus incapable of allowing effective blockage of the aberrant electrical signals or nerve conduction. However, excessively strong contact may probably lead to perforation of the tissue, i.e., an increased safety risk. In order to avoid these issues, existing catheters of this type with force sensors at the distal end can effectively sense the contact strength between the electrode and the vessel wall or tissue. For instance, magnetic position sensors may be equipped in such a catheter to sense contact strength between the distal end thereof and the target organ. However, such sensors suffer from certain limitations in practice, such as tending to give distorted results due to interference from external magnetic fields and limiting other catheter functionalities such as three-dimensional magnetic positioning due to the use of magnetic fields. There are also catheter systems using force-sensitive materials as force sensors for sensing loads on the distal end. Although such systems are good at axial load measurement, they are lack of accuracy in non-axial load measurement. There are still other catheters employing fiber-optic systems for sensing contact forces with the vessel wall or organ, but they are difficult to package and make and expensive and require external electrical signal devices.

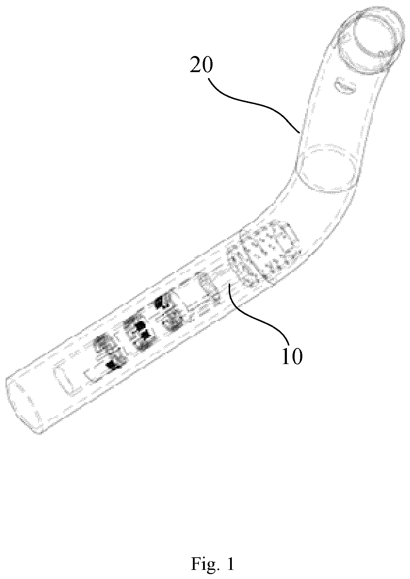

Fig. 1 schematically illustrates a conventional electrophysiology catheter, with a force sensor, passing through an intracardiac guide sheath. As shown in Fig. 1, a guide sheath 20 establishes a channel through which an electrophysiology catheter with a force sensor 10 can be delivered into the body to perform an associated interventional procedure. The electrophysiology catheter may be an ablation catheter, a mapping catheter or another type of electrophysiology catheter.

The electrophysiology catheter includes a distal end at which the force sensor 10 is disposed. The force sensor 10 is configured to obtain the magnitude and direction of a contact force that occurs when the distal end of the catheter is brought into contact with the surface of a vessel wall or tissue. In other words, the force sensor 10 is configured to measure a reaction force in response to the contact force exerted by the catheter's distal end on the vessel wall or tissue.

In practice, in order to guide such electrophysiology catheters to various target sites, distinct guide sheaths 20 are designed with distal ends having different curved shapes. Additionally, such a guide sheath 20 should be constructed to be relatively stiff in order to maintain the designed curved shape while ensuring that a respective electrophysiology catheter can reach the target site. Consequently, as the electrophysiology catheter is guided through the guide sheath 20, the force sensor 10 tends to experience a great deformation under the action of significant forces exerted by the guide sheath 20.

This requires the force sensor 10 to be able to withstand large bending loads. Otherwise, when impacted by a large force, the force sensor 10 may break, leading to failure or a shortened service life of a strain gauge therein due to a load exceeding its measuring range.

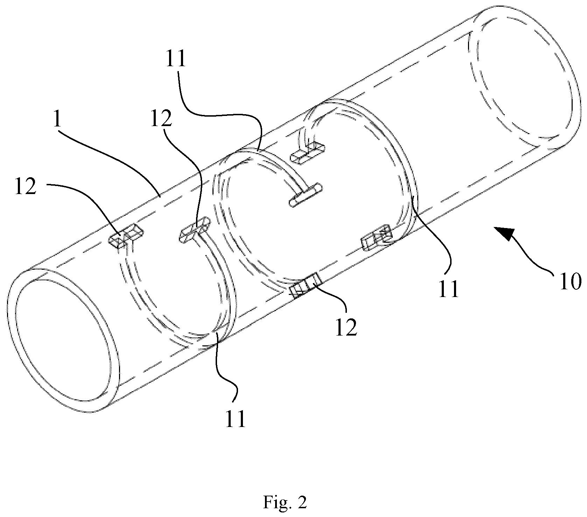

As shown in Fig. 2, the conventional force sensor 10 may include an elastic tube 1 having a wall in which one or more pierced transverse slots 11 (i.e., slots formed by cutting though the wall of the elastic tube 1) are formed in order to enhance elasticity of the elastic tube 1 (especially a metal tube) and amplify any deformation thereof under the action of the transverse slots 11. A strain gauge on the elastic tube 1 may then sense the amplified deformation and outputs an electrical signal indicating the change. Preferably, multiple such transverse slots 11 are formed along different circumferential circles and spaced from one another circumferentially in a staggered manner. That is, orthographic projections of these transverse slots 11 on a single plane are preferably distributed in a circumferentially staggered pattern.

Each transverse slot 11 is an arc-shaped slot cut along a circumference of the wall of the elastic tube 1 and provided at its both opposing ends each with a longitudinal slot 12 for mitigating stress concentration at the opposing ends of the transverse slot 11 and thus increasing its tensile strength.

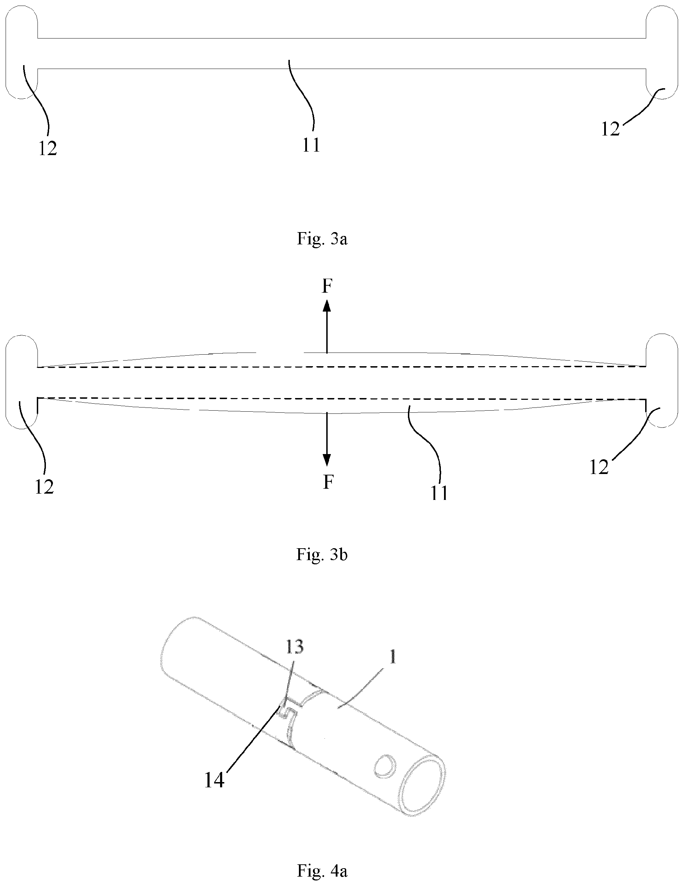

However, as shown in Figs. 3a and 3b, when the elastic tube 1 is stretched, the transverse slot 11 will axially deform at its both axial sides away from the initial positions indicated by dashed lines in the figure, which will still cause the issue of "stress concentration" at the opposing ends. In particular, when the deformation exceeds a certain limit, the transverse slot 11 may crack, or even break, at the opposing ends, leading to permanent failure of the strain gauge due to the excessive strain load exceeding its maximum measuring range.

CN 106 264 719 A discloses an electrophysiological catheter including distal end of catheter provided with pressure transducer including elastic body and foil gauge.

CN 107 496 055 A discloses a heart valve delivery catheter including outer tube assembly and an inner tube component arranged in the outer tube assembly.

### SUMMARY OF THE INVENTION

It is an objective of the present invention is to provide a force sensor and an electrophysiology catheter. The force sensor can be effectively avoided from experiencing any significant impact when the electrophysiology catheter is guided through a sheath. In this way, it is maintained within a predetermined deformation range that prevents breakage of an elastic tube of the sensor and enables an extended service life of any strain gauge therein.

To achieve the above objective, the present invention provides a force sensor as defined in claim 1.

Preferably, the force sensor further comprises at least one second limiting structure disposed between the opposing ends of the transverse slot, each of the at least one second limiting structure comprising a third limiting portion connected to the first wall and a fourth limiting portion connected to the second wall, and
when the transverse slot deforms axially, the third limiting portion and fourth limiting portion are able to responsively move relative to each other until being engaged with each other.

Preferably, the third limiting portion is a male portion with a third external surface and the fourth limiting portion is a female portion with a fourth external surface, the third external surface and the forth external surface being arranged opposite to each other and defining a clearance between the third external surface and the forth external surface, and wherein the third limiting portion is confined within the fourth limiting portion.

Preferably, the first limiting portion, second limiting portion, third limiting portion and fourth limiting portion are of a same shape.

Preferably, the force sensor comprises a plurality of the first limiting structures, and wherein each of the at least one second limiting structure is disposed between adjacent two of the first limiting structures, the third limiting portion is a portion between adjacent two of the first limiting portions thereof and the fourth limiting portion is a gap between adjacent two of the second limiting portions.

Preferably, an external surface of the second limiting portion and an external surface of the third limiting portion are arranged axially opposite to each other and define a clearance between the external surface of the second limiting portion and the external surface of the third limiting portion.

Preferably, the second limiting portion comprises a second vertical portion and a second horizontal portion, and the third limiting portion comprises a third vertical portion and a third horizontal portion, the second vertical portion being connected to the second wall, the third vertical portion being connected to the first wall, the second and third horizontal portions together forming an engagement.

Preferably, the male portions are trapezoidal, L-shaped or T-shaped.

Preferably, the force sensor further comprises two longitudinal slots arranged at the respective opposing ends of the transverse slot.

Preferably, the transverse slot extends in a curved shape on a circumferential surface of the elastic tube.

Preferably, the transverse slot is formed in the elastic tube by laser cutting.

Preferably, the force sensor comprises a plurality of the transverse slots and a plurality of the strain gauges, the transverse slots spaced apart from one another across the elastic tube in an axial direction of the elastic tube and staggered from one another along a circumferential direction of the elastic tube, each of the strain gauges disposed between two opposing ends of a respective one of the transverse slots.

According to the present invention, the force sensor includes an elastic tube and a strain gauge arranged on the elastic tube. A pierced transverse slot is formed in the elastic tube, and at least one first limiting structure is disposed between two opposing ends of the transverse slot. Each first limiting structure includes a first limiting portion connected to a wall of the transverse slot and a second limiting portion connected to the other wall of the transverse slot. In the event of an axial deformation occurring to the transverse slot, the first and second limiting portions will simultaneously move relative to each other. In particular, when the axial deformation reaches a certain level, the two limiting portions will engage with each other. As a result, the transverse slot is maintained within a predetermined deformation range that can avoid breakage of the elastic tube and result in a prolonged service life of the strain gauge.

### BRIEF DESCRIPTION OF THE DRAWINGS

Fig. 1 schematically illustrates a conventional electrophysiology catheter, with a force sensor, passing through an intracardiac guide sheath.
Fig. 2 is a structural schematic of an elastic tube in the conventional force sensor.
Fig. 3a is a structural schematic of a transverse slot in the conventional elastic tube that is not axially deformed.
Fig. 3b is another structural schematic of the transverse slot that is being axially deformed.
Fig. 4a is a schematic perspective view of an elastic tube with an L-shaped limiting structure according to a first embodiment of the present invention.
Fig. 4b is a plan view schematically illustrating the L-shaped limiting structure of Fig. 4a that is arranged at each of opposing ends of a transverse slot.
Fig. 5a is a plan view schematically illustrating a trapezoidal limiting structure at each of opposing ends of a transverse slot according to a second embodiment of the present invention.
Fig. 5b is a schematic illustration of the transverse slot of Fig. 5a that has experienced an axial deformation.
Fig. 6a is a schematic perspective view of a major arc-shaped limiting structure in an elastic tube according to an example not belonging to the present invention.
Fig. 6b is a plan view schematically illustrating the major arc-shaped limiting structure of the Fig. 6a that is arranged at each of opposing ends of a transverse slot.
Fig. 6c is a schematic illustration of the transverse slot of Fig. 6b that has experienced an axial deformation.
Fig. 6d is a plan view schematically illustrating a plurality of major arc-shaped limiting structures at opposing ends of a transverse slot according to an example not belonging to the present invention.
Fig. 7a is a schematic perspective view of a plurality of L-shaped limiting structures in an elastic tube according to a fifth embodiment of the present invention.
Fig. 7b is a schematic plan view partially illustrating the plurality of L-shaped limiting structures of Fig. 7a that are arranged at opposing ends of a transverse slot.
Fig. 7c is a schematic plan view partially illustrating the limiting structures of Fig. 7a that are arranged at the opposing ends of the transverse slot when the transverse slot does not deform at all.
Fig. 7d is a schematic plan view partially illustrating the limiting structures of Fig. 7a that are arranged at the opposing ends of the transverse slot after the transverse slot has experienced a deformation.
Fig. 8 is a schematic perspective view of a plurality of trapezoidal limiting structures in an elastic tube according to a sixth embodiment of the present invention.
Fig. 9 is a diagram schematically illustrating a bending load-bearing performance comparison drawn between elastic tubes respectively adopting a conventional transverse slot and the modified transverse slot of Fig. 7a.

In these figures,
10 denotes a force sensor; 1, an elastic tube; 11, a transverse slot; 111, a first wall; 112, a second wall; 12, a longitudinal slot; 13, a first limiting portion; 14, a second limiting portion; 15, a third limiting portion; 16, a fourth limiting portion; and
20, a guide sheath.

### DETAILED DESCRIPTION

Specific embodiments of the proposed force sensor and electrophysiology catheter will be described in greater detail with reference to the accompanying drawings so that the present invention will become more apparent and readily understood.

Additionally, while the present invention is described in detail with reference to the annexed schematic figures, these figures are presented only for the purpose of facilitating the detailed description of the examples rather than limiting the invention in any sense.

As used herein, the terms "proximal" and "distal" describe relative orientations, positions and directions between elements or actions, viewed by a physician operating the product. Without wishing to be limiting, a "proximal end" usually refers to an end of the product close to the physician during normal operation, while a "distal end" usually refers to an end thereof that enters the patient first. "Axial" and "longitudinal" refer to an axial direction of an elastic tube, while "circumferential" and "transverse" refer to a circumferential direction thereof.

As used in the specification, the singular forms "a," "an" and "the" include plural referents unless the context clearly dictates otherwise. As used in the specification, the term "or" is generally employed in the sense including "and/or" unless the context clearly dictates otherwise.

According to the present invention, in order to enhance breakage resistance of the elastic tube, each transverse slot 11 is maintained within a predetermined deformation range that ensures that the elastic tube 1 will not break. To this end, at least one first limiting structure is added between opposing ends of each transverse slot 11.

In response to an axial deformation of the transverse slot 11, a relative movement will occur in the at least one first limiting structure, and when reaching a limit position, an engagement will occur, defining a maximum deformation amount of the transverse slot 11. In this way, any significant impact can be effectively avoided when the electrophysiology catheter is guided through a sheath, and the elastic tube 1 can be maintained within a predetermined deformation range that prevents breakage of the elastic tube and enables an extended service life of a strain gauge. Generally, such a strain gauge provides a strain measurement range of ±20,000 microstrains. Accordingly, the at least one first limiting structure is generally configured to limit the maximum deformation amount to be not over 20,000 microstrains.

According to the present invention, each first limiting structure may include a first limiting portion and a second limiting portion. The first limiting portion is connected to a first wall of the transverse slot 11. Upon any axial deformation of the first wall, the first limiting portion will experience a synchronous axial displacement. Additionally, the second limiting portion is connected to a second wall of the transverse slot 11. Similarly, when the second wall deforms axially, a synchronous axial displacement will responsively occur to the second limiting portion. The first wall opposes the second wall along a widthwise direction of the transverse slot 11.

Thus, when the elastic tube 1 is stressed and stretched by a force, both the first and second walls will deform accordingly, driving the first and second limiting portions to move relative to (i.e., toward or away from) each other. When the axial load is below a predefined threshold (e.g., 500 g), the relative movement of the first and second limiting portions will be accommodated in a preset clearance without resulting in a contact therebetween. However, when the axial load exceeds the predefined threshold, the first and second limiting portions will move toward or away from each other until the limit position is reached, where they cooperate to form a snap-fit engagement that prevents any of them from further moving. At this point, the transverse slot 11 deforms by a maximum allowable amount under the action of the snap-fit engagement of the first and second limiting portions.

Obviously, in order to control the maximum deformation amount of the transverse slot 11, an axial clearance between the first and second limiting portions is necessary for allowing an axial relative movement between the two limiting portions while ensuring a space for compressive or tensile deformation of the elastic tube 1. Therefore, the maximum deformation amount can be controlled, in general terms, below 20,000 microstrains by suitably setting a dimension of the clearance.

Various examples of the limiting structure according to embodiments of the present invention will be described in greater detail below with reference to Figs. 4a to 9, but the present invention is not limited to these listed examples.

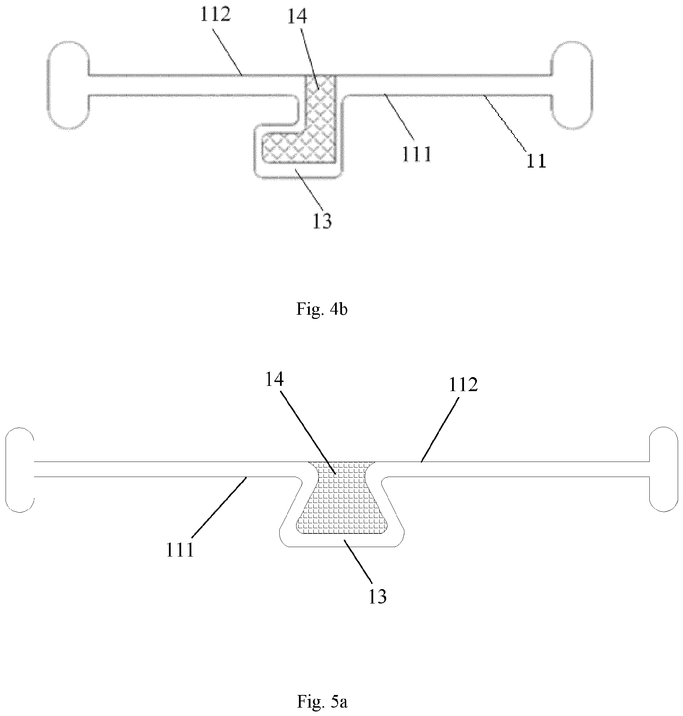

As shown in Figs. 4a and 4b, in a first embodiment, a transverse slot 11 formed in an elastic tube 1 has a first wall 111 and a second wall 112, arranged along a widthwise direction of the slot. The widthwise direction is defined as an axial direction when a center axis of the transverse slot coincides with a center axis of the elastic tube. A first limiting portion 13 is arranged on the first wall 111, while a second limiting portion 14 is arranged on the second wall 112. The first and second limiting portions 13, 14 are both L-shaped portions that match each other in shape. Specifically, the first limiting portion 13 is a female L-shaped portion with a first internal surface, while the second limiting portion 14 is a male L-shaped portion with a second external surface. When the elastic tube 1 is not stressed, the first internal surface and the second external surface oppose each other with a clearance therebetween. In this way, when the elastic tube 1 experiences a force, the second limiting portion 14 is able to move with the transverse slot 11 relative to (i.e., to approach or get away from) the first limiting portion 13 while being always confined within the first limiting portion 13 without dislodging therefrom.

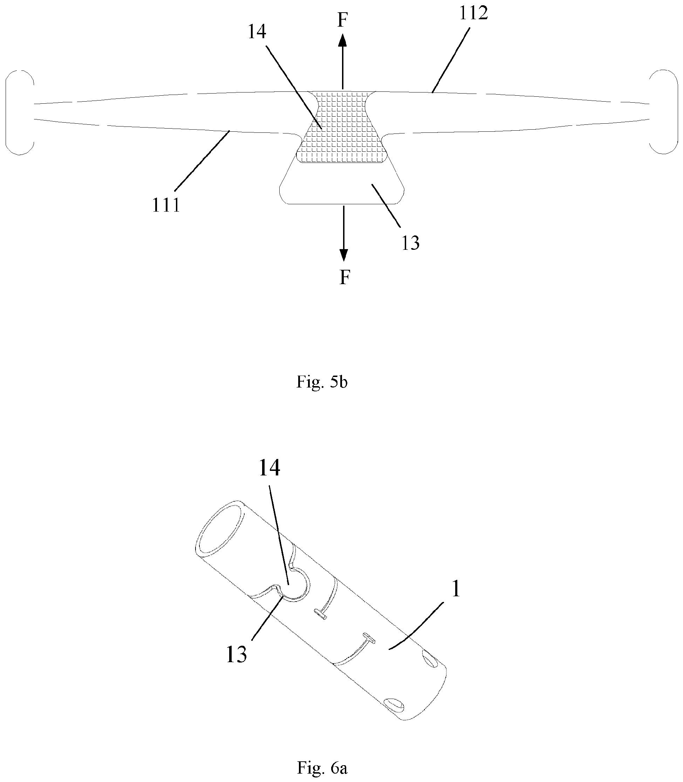

As shown in Figs. 5a and 5b, in a second embodiment, a transverse slot 11 formed in an elastic tube 1 also has a first wall 111 and a second wall 112. A first limiting portion 13 is arranged on the first wall 111, while a second limiting portion 14 is arranged on the second wall 112. In this embodiment, the first limiting portion 13 is a trapezoidal male portion, while the second limiting portion 14 is a trapezoidal female portion. Following the same principles, when the elastic tube 1 is stressed and stretched (as shown in Fig. 5b), the trapezoidal male and female portions will move away from each other until a limit position is reached where a maximum cross-sectional width of the trapezoidal male portion is greater than a minimum cross-sectional width of the trapezoidal female portion (i.e., an opening width), bringing an external surface of the trapezoidal male portion into abutment with an internal surface of the trapezoidal female portion and thus resulting in a snap fit engagement therebetween.

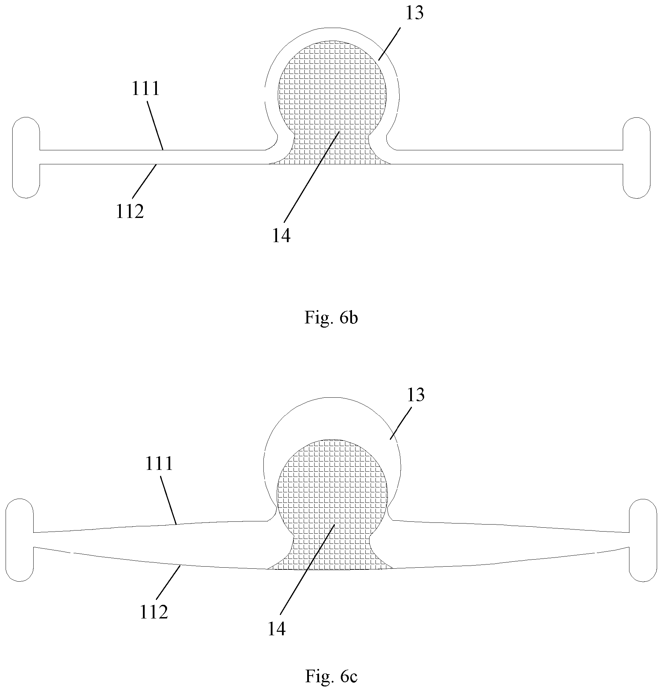

As shown in Figs. 6a to 6c, in an example not belonging to the invention, a transverse slot 11 formed in an elastic tube 1 also has a first wall 111 and a second wall 112. A first limiting portion 13 is arranged on the first wall 111, while a second limiting portion 14 is arranged on the second wall 112. In this embodiment, the first limiting portion 13 is a major arc-shaped female portion, while the second limiting portion 14 is a major arc-shaped male portion. Following the same principles, when the elastic tube 1 is stressed and stretched (as shown in Fig. 6c), the major arc-shaped male and female portions will move away from each other until a limit position is reached where an external surface of the major arc-shaped male portion is brought into close abutment with an internal surface of the major arc-shaped female portion, resulting in a snap fit engagement therebetween, because the major arc-shaped male portion has at least one portion having a cross-sectional width that is greater than an opening width of the major arc-shaped female portion.

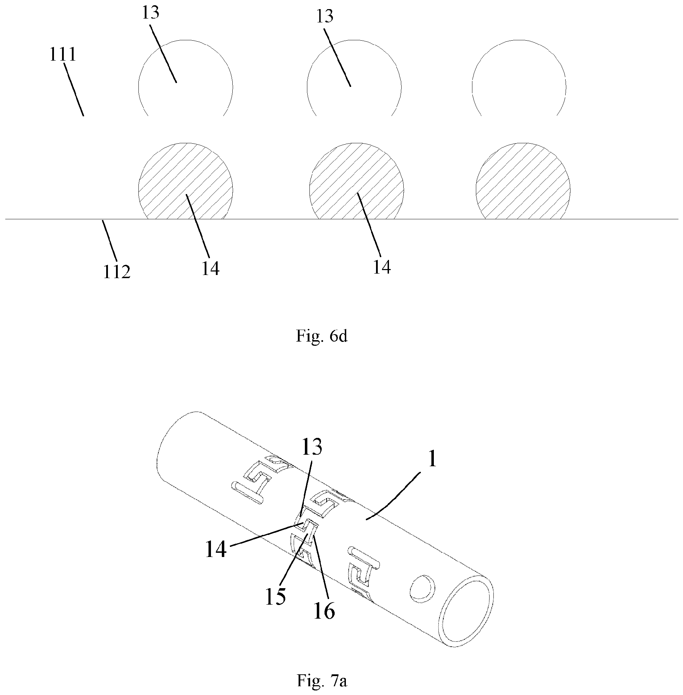

Fig. 6d shows another example not belonging to the invention that is similar to the one previously introduced, except that a plurality of first limiting structures are arranged on opposing ends of a transverse slot 11 and spaced apart from one another. For the sake of brevity, similarities between the two embodiments will not be described herein. The arrangement of the plurality of first limiting structures imparts increased impact resistance and reliability to the elastic tube 1. Even when any of the first limiting structures fails, the remaining one(s) can still provide a limiting effect. In addition, the elastic tube 1 also obtains improved toughness and enhanced sensing ability.

The present invention further provides a fifth embodiment, The fifth embodiment is similar to the first embodiment except that, between adjacent two first limiting structures, a second limiting structure is arranged, which includes a third limiting portion 15 connected to the first wall 111 of the transverse slot 11 and a fourth limiting portion 16 connected to the second wall 112 of the transverse slot 11. For the sake of brevity, similarities between the two embodiments will not be described herein. In response to an axial deformation of the transverse slot 11, the third and fourth limiting portions 15, 16 can move relative to each other until they are engaged with each other.

As shown in Fig. 7a, the third limiting portion 15 is a male portion with a third external surface, while the fourth limiting portion 16 is a female portion with a fourth internal surface that opposes the third internal surface with a clearance therebetween. Additionally, the third limiting portion 15 is confined within the fourth limiting portion 16. In this embodiment, the first limiting portion 13, second limiting portion 14, third limiting portion 15 and fourth limiting portions 16 are in the same shape. In fact, the third limiting portion 15 is composed of a portion between the adjacent two first limiting portions 13, while the fourth limiting portion 16 is composed of a gap between the adjacent two second limiting portions 14.

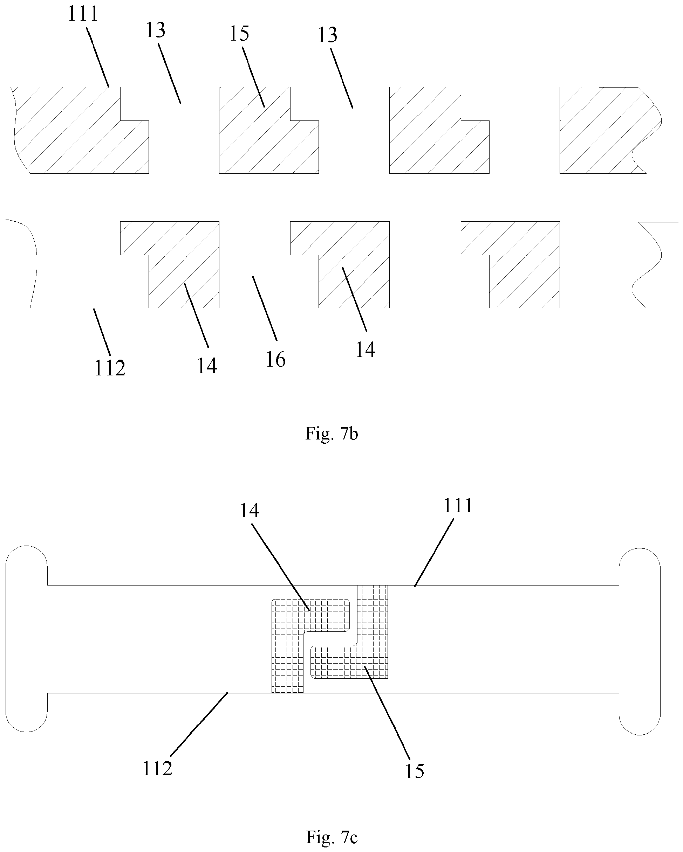

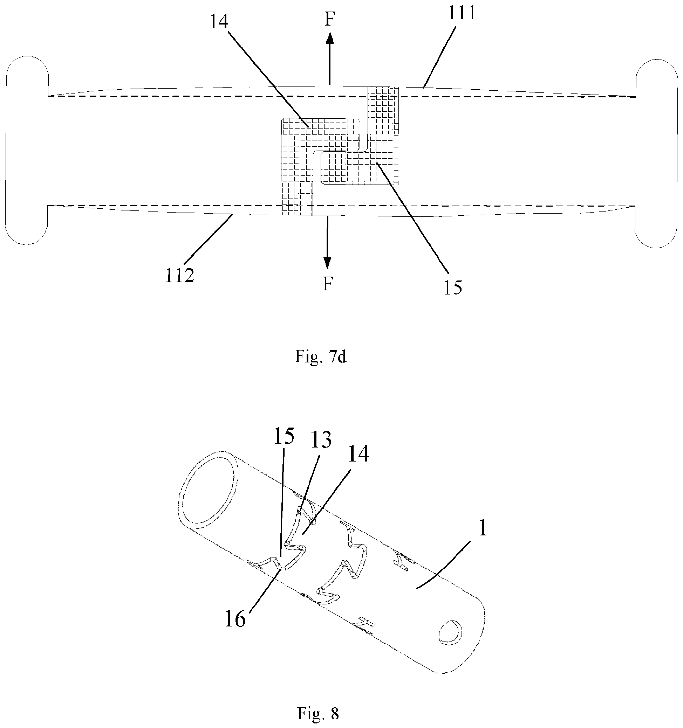

More specifically, as shown in Figs. 7c and 7d, each second limiting portion 14 has a second vertical portion and a second horizontal portion, and the third limiting portion 15 has a third vertical portion and a third horizontal portion. The second vertical portion is connected to the second wall 112, while the third vertical portion is connected to the first wall 111. The second horizontal portion axially opposes the third horizontal portion, thus forming a snap mechanism. Here, mutually-facing surfaces of the second and third horizontal portions are referred to as their "internal surfaces", and the respective opposite surfaces as their "external surfaces". Initially, there is a clearance between the internal surfaces of the two horizontal portions (as shown in Fig. 7c), which allows a relative movement (generally, an approaching movement) of the two limiting portions. As an associated deformation increases, the horizontal portions of the two limiting portions will be finally engaged with each other (as shown in Fig. 7d). That is, the internal surfaces of the two horizontal portions are brought into engagement with each other, which prevents the two horizontal portions from further moving toward each other anymore. In other words, after an approaching movement is made from the positions shown in Fig. 7c, the horizontal portions of the two limiting portions 14, 15 reach a limit position, where their internal surfaces abut against each other, thus defining a maximum deformation limit of the transverse slot 11. In other embodiments, the horizontal portions of the two limiting portions 14, 15 may also move away from each other from the positions as shown in Fig. 7c until the external surface of the second horizontal portion in the second limiting portion 14 abuts against the internal surface of the first limiting portion 13 (as shown in Fig. 7b) so that their further relative movement is again disallowed.

Fig. 8 shows a sixth embodiment that is similar to the fifth embodiment except that the first limiting portion 13, second limiting portion 14, third limiting portion 15 and fourth limiting portion 16 are all trapezoidal. For the sake of brevity, similarities between the two embodiments will not be described herein. In case of the first limiting portion 13, second limiting portion 14, third limiting portion 15 and fourth limiting portion 16 being of the same shape, the two male limiting structures will be identically strong, preventing either of them from earlier breakage due to lower strength. In addition, the identical shape allows a maximized contact area between them, preventing the occurrence of insufficient contact therebetween. Further, relatively speaking, the plurality of L-shaped limiting structures can provide a more secure engagement than those of other shapes such as the trapezoidal or major arc shape.

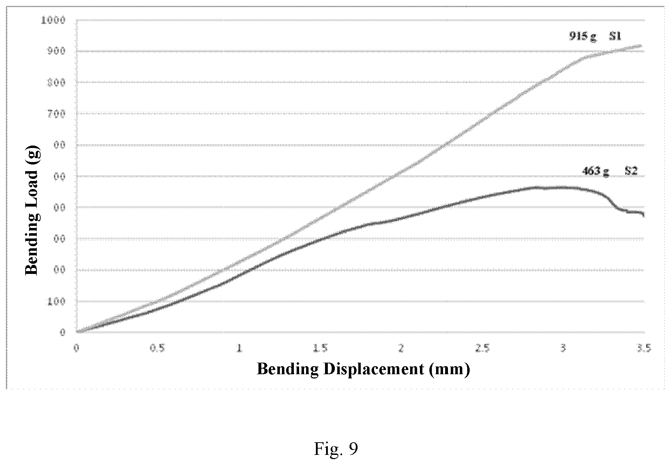

Experimental results demonstrate that, a conventional transverse slot 11 without any limiting structure arranged between its opposing ends can withstand a maximum bending load of 463 g and the elastic tube 1 will break upon any increase in the load, as indicated by the curve s2 in Fig. 9. In spite of this, when advanced through a guide sheath 20, it is very likely for a force sensor 10 to experience a load of 500 g or greater when the advancement is too fast or an inappropriate driving force is applied. By contrast, a maximum bending load that a transverse slot 11 adopting the structure of Figs. 7a and 7b can withstand is greater than 900 g, as indicated by the curve s1 in Fig. 9, undoubtedly meeting the practical requirements. Fig. 9 is a diagram schematically illustrating a bending load-bearing performance comparison between the elastic tubes respectively adopting the conventional transverse slot and the modified transverse slot of Fig. 7a, in which the horizontal axis represents the bending displacement (measured in mm) and in which the vertical axis represents the bending load (measured in gram).

In any of the above embodiments, the transverse slot 11 may extend in a curved shape on the circumferential surface of the elastic tube 1. That is, the transverse slot 11 appears curved in a front view of the elastic tube 1, and arc-shaped in a top view of the elastic tube 1. Preferably, the transverse slot is formed in the elastic tube 1 by a laser cutting. The force sensor 10 further includes strain gauge(s) arranged on the outer wall of the elastic tube 1. The number of the strain gauge(s) corresponds to the number of the transverse slot(s). Each strain gauge may be provided on either a pierced or non-pierced section of the elastic tube 1. Here, the term "pierced section" refers to a portion encompassing one of the transverse slot(s) (i.e., the strain gauge can cover the transverse slot 11), whereas the term "non-pierced section" refers to a portion of the elastic tube 1 that is not pierced. Preferably, each strain gauge is arranged on a non-pierced section of the elastic tube 1. More preferably, a plurality of the transverse slots 11 are axially spaced apart from one another across the elastic tube 1 and staggered from one another along the circumferential direction thereof, with each strain gauge preferably disposed between opposing ends of the respective transverse slot.

At last, while a few preferred embodiments of the present invention have been described above, the present invention is not limited to the scope of these disclosed embodiments. For example, in case of a plurality of limiting structures being provided, all of them may have the same structure, or one or more of them may have a different structure.

According to embodiments of the present invention, the force sensor includes an elastic tube and a strain gauge arranged on the elastic tube. A transverse slot is formed in the elastic tube, and at least one first limiting structure is disposed between opposing ends of the transverse slot. Each first limiting structure includes a first limiting portion connected to a wall of the transverse slot and a second limiting portion connected to the other wall of the transverse slot. In the event of an axial deformation occurring to the transverse slot, the first and second limiting portions will responsively move relative to each other. In particular, when the axial deformation reaches a certain level, the two limiting portions will engage with each other. As a result, the transverse slot is maintained within a predetermined deformation range that can avoid breakage of the elastic tube and result in a prolonged service life of the strain gauge. In particular, adding one or more such limiting structures will not lead to increased structural complexity or impaired overall appearance of the elastic tube, and the limiting structures themselves are very easy to form and suitable for industrial production and use.

## Claims

1. A force sensor (10), comprising an elastic tube (1) and a strain gauge arranged on the elastic tube (1), wherein:
the force sensor (10) further comprises a pierced transverse slot (11) formed in the elastic tube (1), the transverse slot (11) having a first wall (111) and a second wall (112);
the force sensor (10) **characterised in that** it further comprises at least one first limiting structure disposed between two opposing ends of the transverse slot (11), each of the at least first limiting structure comprising a first limiting portion (13) and a second limiting portion (14), the first limiting portion (13) being connected to the first wall (111), the second limiting portion (14) being connected to the second wall (112); the first limiting portion (13) is a female portion with a first internal surface and the second limiting portion (14) is a male portion with a second external surface, the first internal surface and the second external surface being arranged opposite to each other and defining a clearance between the first internal surface and the second external surface, and wherein the second limiting portion (14) is confined within the first limiting portion (13); and
when the elastic tube is stretched by a force and the transverse slot (11) deforms in an axial direction of the elastic tube (1), the first limiting portion (13) and the second limiting portion (14) are able to responsively move away from each other in the axial direction until being engaged with each other;
wherein the first limiting portion (13) and second limiting portion (14) are both L-shaped portions that match each other in shape; or the first limiting portion (13) is a trapezoidal male portion, and the second limiting portion (14) is a trapezoidal female portion.

2. The force sensor (10) of claim 1, wherein the force sensor (10) comprises a plurality of the first limiting structures spaced apart from one another across a same circumferential surface.

3. The force sensor (10) of claim 1, further comprising at least one second limiting structure disposed between the opposing ends of the transverse slot (11), each of the at least one second limiting structure comprising a third limiting portion (15) connected to the first wall (111) and a fourth limiting portion (16) connected to the second wall (112), and
when the transverse slot (11) deforms axially, the third limiting portion (15) and fourth limiting portion (16) are able to responsively move relative to each other until being engaged with each other.

4. The force sensor (10) of claim 3, wherein the third limiting portion (15) is a male portion with a third external surface and the fourth limiting portion (16) is a female portion with a fourth external surface, the third external surface and the forth external surface being arranged opposite to each other and defining a clearance between the third external surface and the forth external surface, and wherein the third limiting portion (15) is confined within the fourth limiting portion (16).

5. The force sensor (10) of claim 3, wherein the first limiting portion (13), second limiting portion (14), third limiting portion (15) and fourth limiting portion (16) are of a same shape.

6. The force sensor (10) of claim 5, wherein the force sensor (10) comprises a plurality of the first limiting structures, and wherein each of the at least one second limiting structure is disposed between adjacent two of the first limiting structures, the third limiting portion (15) is a portion between adjacent two of the first limiting portions (13) thereof and the fourth limiting portion (16) is a gap between adjacent two of the second limiting portions (14).

7. The force sensor (10) of claim 5, wherein an external surface of the second limiting portion (14) and an external surface of the third limiting portion (15) are arranged axially opposite to each other and define a clearance between the external surface of the second limiting portion (14) and the external surface of the third limiting portion (15).

8. The force sensor (10) of claim 7, wherein the second limiting portion (14) comprises a second vertical portion and a second horizontal portion, and the third limiting portion (15) comprises a third vertical portion and a third horizontal portion, the second vertical portion being connected to the second wall (112), the third vertical portion being connected to the first wall (111), the second and third horizontal portions together forming an engagement.

9. The force sensor (10) of claim 1, wherein the force sensor (10) further comprises two longitudinal slots (12) arranged at the respective opposing ends of the transverse slot (11).

10. The force sensor (10) of claim 1, wherein the transverse slot (11) extends in a curved shape on a circumferential surface of the elastic tube (1); or.
the transverse slot (11) is formed in the elastic tube (1) by laser cutting.

11. The force sensor (10) of claim 1, wherein the force sensor (10) comprises a plurality of the transverse slots (11) and a plurality of the strain gauges, the transverse slots (11) spaced apart from one another across the elastic tube (1) in the axial direction of the elastic tube (1) and staggered from one another along a circumferential direction of the elastic tube (1), each of the strain gauges disposed between two opposing ends of a corresponding one of the transverse slots (11).

12. An electrophysiology catheter, comprising a distal end and a force sensor (10) as defined in any one of claims 1 to 11, the force sensor (10) being disposed at the distal end.

## Patentansprüche

1. Kraftsensor (10), umfassend ein elastisches Rohr (1) und einen auf dem elastischen Rohr (1) angeordneten Dehnungsmessstreifen, wobei:
der Kraftsensor (10) ferner einen durchbohrten Querschlitz (11) umfasst, der in dem elastischen Rohr (1) gebildet ist, wobei der Querschlitz (11) eine erste Wand (111) und eine zweite Wand (112) aufweist;
der Kraftsensor (10) **dadurch gekennzeichnet ist, dass** er ferner mindestens eine erste Begrenzungsstruktur umfasst, die zwischen zwei gegenüberliegenden Enden des Querschlitzes (11) angeordnet ist, wobei jede der mindestens ersten Begrenzungsstrukturen einen ersten Begrenzungsabschnitt (13) und einen zweiten Begrenzungsabschnitt (14) umfasst, wobei der erste Begrenzungsabschnitt (13) mit der ersten Wand (111) verbunden ist, wobei der zweite Begrenzungsabschnitt (14) mit der zweiten Wand (112) verbunden ist; wobei der erste Begrenzungsabschnitt (13) ein weiblicher Abschnitt mit einer ersten Innenfläche und der zweite Begrenzungsabschnitt (14) ein männlicher Abschnitt mit einer zweiten Außenfläche ist, wobei die erste Innenfläche und die zweite Außenfläche einander gegenüberliegend angeordnet sind und einen Abstand zwischen der ersten Innenfläche und der zweiten Außenfläche definieren, und wobei der zweite Begrenzungsabschnitt (14) innerhalb des ersten Begrenzungsabschnitts (13) eingeschlossen ist; und
wenn das elastische Rohr durch eine Kraft gedehnt wird und sich der Querschlitz (11) in einer axialen Richtung des elastischen Rohrs (1) verformt, der erste Begrenzungsabschnitt (13) und der zweite Begrenzungsabschnitt (14) ausgebildet sind, sich in der axialen Richtung reaktiv voneinander weg zu bewegen, bis sie miteinander eingegriffen werden;
wobei der erste Begrenzungsabschnitt (13) und der zweite Begrenzungsabschnitt (14) beide L-förmige Abschnitte sind, deren Form zueinander passt; oder wobei der erste Begrenzungsabschnitt (13) ein trapezförmiger männlicher Abschnitt und der zweite Begrenzungsabschnitt (14) ein trapezförmiger weiblicher Abschnitt ist.

2. Kraftsensor (10) nach Anspruch 1, wobei der Kraftsensor (10) eine Vielzahl der ersten Begrenzungsstrukturen umfasst, die über eine gleiche Umfangsfläche voneinander beabstandet sind.

3. Kraftsensor (10) nach Anspruch 1, ferner umfassend mindestens eine zweite Begrenzungsstruktur, die zwischen den gegenüberliegenden Enden des Querschlitzes (11) angeordnet ist, wobei jede der mindestens einen zweiten Begrenzungsstrukturen einen dritten Begrenzungsabschnitt (15), der mit der ersten Wand (111) verbunden ist, und einen vierten Begrenzungsabschnitt (16) umfasst, der mit der zweiten Wand (112) verbunden ist, und
wobei, wenn sich der Querschlitz (11) axial verformt, der dritte Begrenzungsabschnitt (15) und vierter Begrenzungsabschnitt (16) ausgebildet sind, sich relativ zueinander reaktiv zu bewegen, bis sie miteinander eingegriffen werden.

4. Kraftsensor (10) nach Anspruch 3, wobei der dritte Begrenzungsabschnitt (15) ein männlicher Abschnitt mit einer dritten Außenfläche und der vierte Begrenzungsabschnitt (16) ein weiblicher Abschnitt mit einer vierten Außenfläche ist, wobei die dritte Außenfläche und die vierte Außenfläche einander gegenüberliegend angeordnet sind und einen Abstand zwischen der dritten Außenfläche und der vierten Außenfläche definieren, und wobei der dritte Begrenzungsabschnitt (15) innerhalb des vierten Begrenzungsabschnitts (16) eingeschlossen ist.

5. Kraftsensor (10) nach Anspruch 3, wobei der erste Begrenzungsabschnitt (13), zweite Begrenzungsabschnitt (14), dritte Begrenzungsabschnitt (15) und vierter Begrenzungsabschnitt (16) eine gleiche Form aufweisen.

6. Kraftsensor (10) nach Anspruch 5, wobei der Kraftsensor (10) eine Vielzahl der ersten Begrenzungsstrukturen umfasst, und wobei jede der mindestens einen zweiten Begrenzungsstrukturen zwischen zwei benachbarten der ersten Begrenzungsstrukturen angeordnet ist, wobei der dritte Begrenzungsabschnitt (15) ein Abschnitt zwischen zwei davon benachbarten der ersten Begrenzungsabschnitte (13) ist, und wobei der vierte Begrenzungsabschnitt (16) ein Spalt zwischen zwei benachbarten der zweiten Begrenzungsabschnitte (14) ist.

7. Kraftsensor (10) nach Anspruch 5, wobei eine Außenfläche des zweiten Begrenzungsabschnitts (14) und eine Außenfläche des dritten Begrenzungsabschnitts (15) einander axial gegenüberliegend angeordnet sind und einen Abstand zwischen der Außenfläche des zweiten Begrenzungsabschnitts (14) und der Außenfläche des dritten Begrenzungsabschnitts (15) definieren.

8. Kraftsensor (10) nach Anspruch 7, wobei der zweite Begrenzungsabschnitt (14) einen zweiten vertikalen Abschnitt und einen zweiten horizontalen Abschnitt umfasst, und wobei der dritte Begrenzungsabschnitt (15) einen dritten vertikalen Abschnitt und einen dritten horizontalen Abschnitt umfasst, wobei der zweite vertikale Abschnitt mit der zweiten Wand (112) verbunden ist, wobei der dritte vertikale Abschnitt mit der ersten Wand (111) verbunden ist, wobei der zweite und der dritte horizontale Abschnitt zusammen einen Eingriff bilden.

9. Kraftsensor (10) nach Anspruch 1, wobei der Kraftsensor (10) ferner zwei Längsschlitze (12) umfasst, die an den jeweils gegenüberliegenden Enden des Querschlitzes (11) angeordnet sind.

10. Kraftsensor (10) nach Anspruch 1, wobei der Querschlitz (11) in einer gekrümmten Form auf einer Umfangsfläche des elastischen Rohrs (1) verläuft; oder
wobei der Querschlitz (11) durch Laserschneiden in dem elastischen Rohr (1) geformt ist.

11. Kraftsensor (10) nach Anspruch 1, wobei der Kraftsensor (10) eine Vielzahl von Querschlitzen (11) und eine Vielzahl von Dehnungsmessstreifen umfasst, wobei die Querschlitze (11) in axialer Richtung des elastischen Rohrs (1) über das elastische Rohr (1) voneinander beabstandet sind und entlang einer Umfangsrichtung des elastischen Rohrs (1) zueinander versetzt sind, wobei jeder der Dehnungsmessstreifen zwischen zwei gegenüberliegenden Enden eines entsprechenden der Querschlitze (11) angeordnet ist.

12. Elektrophysiologiekatheter, umfassend ein distales Ende und einen Kraftsensor (10) nach einem der Ansprüche 1 bis 11, wobei der Kraftsensor (10) an dem distalen Ende angeordnet ist.

## Revendications

1. Capteur de force (10), comprenant un tube élastique (1) et une jauge de contrainte disposée sur le tube élastique (1), dans lequel :
le capteur de force (10) comprend en outre une fente transversale percée (11) formée dans le tube élastique (1), la fente transversale (11) comportant une première paroi (111) et une seconde paroi (112) ;
le capteur de force (10) **caractérisé en ce qu'**il comprend en outre au moins une première structure de limitation disposée entre deux extrémités opposées de la fente transversale (11), chacune des au moins premières structures de limitation comprenant une première partie de limitation (13) et une seconde partie de limitation (14), la première partie de limitation (13) étant reliée à la première paroi (111), la seconde partie de limitation (14) étant reliée à la seconde paroi (112) ; la première partie de limitation (13) est une partie femelle avec une première surface interne et la seconde partie de limitation (14) est une partie mâle avec une seconde surface externe, la première surface interne et la seconde surface externe étant disposées opposées l'une à l'autre et définissant un jeu entre la première surface interne et la seconde surface externe, et dans lequel la seconde partie de limitation (14) est confinée à l'intérieur de la première partie de limitation (13) ; et
lorsque le tube élastique est étiré par une force et que la fente transversale (11) se déforme dans une direction axiale du tube élastique (1), la première partie de limitation (13) et la deuxième partie de limitation (14) sont capables de s'éloigner en réponse l'une de l'autre dans la direction axiale jusqu'à être engagées l'une avec l'autre ;
dans lequel la première partie de limitation (13) et la seconde partie de limitation (14) sont toutes deux des parties en forme de L qui correspondent l'une à l'autre en forme ; ou la première partie de limitation (13) est une partie mâle trapézoïdale, et la seconde partie de limitation (14) est une partie femelle trapézoïdale.

2. Capteur de force (10) selon la revendication 1, dans lequel le capteur de force (10) comprend une pluralité de premières structures de limitation espacées les unes des autres sur une même surface circonférentielle.

3. Capteur de force (10) selon la revendication 1, comprenant en outre au moins une seconde structure de limitation disposée entre les extrémités opposées de la fente transversale (11), chacune de la ou des secondes structures de limitation comprenant une troisième partie de limitation (15) reliée à la première paroi (111) et une quatrième partie de limitation (16) relié à la deuxième paroi (112) et
lorsque la fente transversale (11) se déforme axialement, la troisième partie de limitation (15) et quatrième partie limitative (16) sont capables de se déplacer de manière réactive les uns par rapport aux autres jusqu'à ce qu'ils soient engagés l'un avec l'autre.

4. Capteur de force (10) selon la revendication 3, dans lequel la troisième partie de limitation (15) est une partie mâle avec une troisième surface externe et la quatrième partie de limitation (16) est une partie femelle avec une quatrième surface externe, la troisième surface externe et la quatrième surface externe étant disposées en face l'une de l'autre et définissant un espace libre entre la troisième surface externe et la quatrième surface externe, et dans lequel la troisième partie de limitation (15) est confinée à l'intérieur de la quatrième partie de limitation (16).

5. Capteur de force (10) selon la revendication 3, dans lequel le premier partie limitative (13), deuxième partie limitative (14), troisième la partie limitative (15) et la quatrième partie limitative (16) sont de même forme.

6. Capteur de force (10) selon la revendication 5, dans lequel le capteur de force (10) comprend une pluralité de premières structures de limitation, et dans lequel chacune de la ou des deuxièmes structures de limitation est disposée entre deux structures de limitation adjacentes parmi les premières structures de limitation, la troisième partie de limitation (15) est une partie entre deux parties adjacentes des premières parties limitatives (13) de celle-ci et la quatrième partie limitative (16) est un espace entre deux parties adjacentes des secondes parties limitatives (14).

7. Capteur de force (10) selon la revendication 5, dans lequel une surface externe de la seconde partie de limitation (14) et une surface externe de la troisième partie de limitation (15) sont disposées axialement en face l'une de l'autre et définissent un jeu entre la surface externe de la deuxième partie de limitation (14) et la surface externe de la troisième partie de limitation (15).

8. Capteur de force (10) selon la revendication 7, dans lequel la deuxième partie de limitation (14) comprend une deuxième partie verticale et une deuxième partie horizontale, et la troisième partie de limitation (15) comprend une troisième partie verticale et une troisième partie horizontale, la deuxième partie verticale étant reliée à la deuxième paroi (112), la troisième partie verticale étant reliée à la première paroi (111), les deuxième et troisième parties horizontales formant ensemble un engagement.

9. Capteur de force (10) selon la revendication 1, dans lequel le capteur de force (10) comprend en outre deux fentes longitudinales (12) disposées aux extrémités opposées respectives de la fente transversale (11).

10. Capteur de force (10) selon la revendication 1, dans lequel la fente transversale (11) s'étend sous une forme incurvée sur une surface circonférentielle du tube élastique (1) ; ou la fente transversale (11) est formée dans le tube élastique (1) par découpe laser.

11. Capteur de force (10) selon la revendication 1, dans lequel le capteur de force (10) comprend une pluralité de fentes transversales (11) et une pluralité de jauges de contrainte, les fentes transversales (11) étant espacées les unes des autres à travers le tube élastique (1) dans la direction axiale du tube élastique (1) et décalées les unes des autres le long d'une direction circonférentielle du tube élastique (1), chacune des jauges de contrainte étant disposée entre deux extrémités opposées d'une fente transversale correspondante (11).

12. Cathéter d'électrophysiologie, comprenant une extrémité distale et un capteur de force (10) tel que défini dans l'une quelconque des revendications 1 à 11, le capteur de force (10) étant disposé à l'extrémité distale.
